# EUROPÄISCHE PATENTANMELDUNG

(11) **EP 1 382 369 A1**
(43) Veröffentlichungstag der Anmeldung: **21.01.2004**
(21) Anmeldenummer: 02016184.0
(22) Anmeldetag: 20.07.2002
(51) Int. Cl.: A61N 1/39

(54) **Vorrichtung zur Elektrotherapie und Verfahren zum Testen und Betreiben einer solchen Vorrichtung**

(71) Anmelder: Schiller AG, 6340 Baar (CH)
(72) Erfinder: Schiller, Alfred, 8914 Aeugst a. A. (CH); Schmid, Johann-Jakob, 8911 Rifferswil (CH); Cansell, Albert Dr., 67160 Wissembourg (FR)
(74) Vertreter: Müller, Christoph Emanuel

(57) **Zusammenfassung**

Eine Vorrichtung zur Elektrotherapie weist eine Prüfschaltung auf. Die Prüfschaltung dient zum Überprüfen der Funktionen der Vorrichtung in einer Testroutine oder zum Abfragen des Status von Komponenten der Vorrichtung (1). Die Vorrichtung (1) ist mit Kommunikationsmitteln (3) versehen . Die Kommunikationsmittel (3) wirken mit der Prüfschaltung zusammen und dienen zum Übermitteln von Daten, beispielsweise dem Resultat einer Testroutine an einen Empfänger (E).

## Beschreibung

Die Erfindung betrifft eine Vorrichtung zur Elektrotherapie, insbesondere einen tragbaren Defibrillator sowie ein Verfahren zum Testen und/oder Betreiben einer solchen Vorrichtung mit den Merkmalen des Oberbegriffs der unabhängigen Patentansprüche.

Zur Elektrotherapie bei Herzproblemen werden häufig Defibrillatoren eingesetzt. Krankenhäuser oder Rettungssanitäter sind standardmässig mit solchen Geräten ausgerüstet. Modernere Geräte ermöglichen dank Elektronik eine Benutzerführung und das Ausgeben von Benutzerinstruktionen. Eine Bedienung von solchen Geräten ist deshalb auch durch nicht spezifisch ausgebildete Personen möglich. Es wird deshalb angestrebt, solche einfach einsetzbaren Defibrillatoren an öffentlich zugänglichen Orten, beispielsweise in Bahnhöfen, öffentlichen Gebäuden oder Firmengebäuden anzubringen. Damit soll erreicht werden, dass eine Elektrotherapie bei einem Patienten sofort vorgenommen werden kann, auch wenn ein Rettungssanitäter noch nicht anwesend oder noch nicht avisiert ist. Ein Problem bei solchen Defibrillatoren besteht aber darin, dass üblicherweise das Gerät kaum je benutzt wird. Es ist deshalb unsicher, ob im Ernstfall das Gerät noch funktionstüchtig ist. Beispielsweise könnte es sein, dass die Leistung der Stromquelle, typischerweise eine Batterie oder ein Akkumulator, nicht mehr ausreicht. Es wäre auch denkbar, dass technische Defekte aufgrund von Korrosion, Nagetierbiss oder Erschütterungen auftreten. Es ist deshalb bei solchen Geräten erforderlich, dass - ähnlich wie beispielsweise bei Feuerlöschern - eine Inspektion vorgenommen wird. Solche Inspektionen sind aber aufwendig. Auch können sie durch technisch unkundiges Personal nicht ohne weiteres vorgenommen werden.

Es ist bereits bekannt, Defibrillatoren so auszubilden, dass automatisch in regelmässigen Abständen eine Testroutine durchgeführt wird. Das Resultat der Testroutine wird dem Benutzer visuell oder akustisch dargestellt. Solche Vorrichtungen sind beispielsweise in US 6 016 059, US 5 800 460, US 5 899 925, US 5 579 234 oder US 5 591 213 beschrieben. Mit dem darin beschriebenen Selbsttest kann zwar grundsätzlich auch durch unkundiges Personal festgestellt werden, ob das Gerät betriebsbereit ist. Eine regelmässige Inspektion ist aber trotzdem erforderlich, was zu hohen Wartungskosten führt.

Ein weiterer Nachteil bei solchen öffentlich zugänglich platzierten Geräten besteht darin, dass aufgrund von Vandalismus Geräte beschädigt oder entwendet werden könnten, sodass im Ernstfall kein Gerät mehr zur Verfügung steht. Ein weiterer Nachteil bei bekannten Geräten besteht darin, dass häufig bei einem Versuch der Elektrotherapie durch medizinisch nicht geschultes Personal vergessen wird, parallel einen Rettungssanitäter oder Arzt zu avisieren. Auch wenn eine Elektrotherapie durch unkundiges Personal erfolgreich vorgenommen werden kann, sollte in jedem Fall medizinisch geschultes Personal hinzugezogen werden.

Ein weiteres Problem bei solchen bekannten Geräten besteht auch darin, dass bei einem Totalausfall des Gerätes die Testroutine ebenfalls nicht durchgeführt wird.

Es ist deshalb eine Aufgabe der vorliegenden Erfindung, die Nachteile des Bekannten zu vermeiden, insbesondere also eine Vorrichtung zur Elektrotherapie zu schaffen, welche bei geringem Wartungs- und Kontrollaufwand jederzeit eine sichere Funktionsweise der Vorrichtung ermöglicht und daher sicherstellt, dass im Ernstfall zuverlässig eine Elektrotherapie durchgeführt werden kann.

Erfindungsgemäss werden diese Aufgaben mit einer Vorrichtung und mit einem Verfahren mit den Merkmalen des kennzeichnenden Teils der unabhängigen Patentansprüche gelöst.

Die erfindungsgemässe Vorrichtung zur Elektrotherapie ist typischerweise als tragbarer Defibrillator ausgebildet. Der Defibrillator ist in an sich bekannter Weise mit Elektroden, Stromquelle und einer vorzugsweise elektronischen Kontrollund Steuereinrichtung versehen. Die Vorrichtung ist mit einer Prüfschaltung, insbesondere mit einem Testschaltkreis versehen. Der Testschaltkreis dient beispielsweise zum Überprüfen der Funktionen der Vorrichtung in einer Testroutine. Solche Testroutinen sind ebenfalls bereits bekannt. Erfindungsgemäss weist die Vorrichtung Kommunikationsmittel zur Datenübertragung auf. Die Kommunikationsmittel sind mit der Prüfschaltung gekoppelt oder koppelbar. Mittels der Kommunikationsmittel können Resultate und/oder der Ablauf der Testroutine oder andere von der Prüferschaltung bestimmte Resultate einem Empfänger übermittelt werden. Der Empfänger ist typischerweise ein Servicecenter eines Herstellers von solchen Geräten oder eine mit der Wartung der Geräte beauftragte Firma. Es ist aber auch denkbar, dass der Empfänger ein Notarzt oder ein Krankenhaus ist. In bestimmten Fällen könnte der Empfänger auch eine verantwortliche Person der Organisation sein, welche die Vorrichtung betreibt, beispielsweise ein verantwortlicher Arzt einer Eisenbahngesellschaft oder eine Betriebsfeuerwehr.

Es ist denkbar, beliebige bekannte Kommunikationsmittel einzusetzen. So kann die Vorrichtung beispielsweise über eine Telefonleitung mit dem öffentlichen Telefonnetz verbunden werden. Bevorzugt ist die Vorrichtung mit einem GSM-Modul versehen, welches zur drahtlosen Datenübermittlung zwischen den Kommunikationsmitteln und dem Empfänger dient, wobei ebenfalls öffentliche Telefonnetze verwendet werden. Es sind aber auch andere Kommunikationsmittel, beispielsweise UMTS-Telefone oder lokale Funknetzwerke denkbar.

Gemäss einer ersten bevorzugten Ausführungsform der Erfindung ist der Testschaltkreis zum regelmässigen, insbesondere periodischen Durchführen der Testroutine ausgebildet. Die Testroutine kann beispielsweise alle zwei Wochen durchgeführt werden. Es ist aber auch denkbar, die Testroutine früher auszulösen, wenn beispielsweise besondere Umgebungsumstände erfasst werden. Ausserdem ist es alternativ auch denkbar, die Vorrichtung von der Zentrale her anzusprechen. Beispielsweise kann eine Testroutine durch Anruf von einer Zentrale her ausgelöst werden. In diesem Zusammenhang können beispielsweise von der Zentrale periodisch Testroutinen ausgelöst werden. Es ist auch denkbar, die Testroutine bei besonderen Vorkommnissen, beispielsweise bei sehr kalten Temperaturen ausserplanmässig durchzuführen.

So kann beispielsweise eine über längere Zeiten tiefe Temperatur dazu führen, dass die Batterie vorzeitig nicht mehr ausreichende Kapazität zum Durchführen einer Elektrotherapie aufweist. Bei solchen Umgebungsbedingungen kann eine Testroutine vorzeitig ausgelöst werden. Regelmässig heisst im vorliegenden Fall gemäss einer bestimmten Regel. Unter einer regelmässigen Durchführung der Testroutine wird beispielsweise auch das Durchführen der Testroutine in nicht gleichbleibenden Zeitabständen, beispielsweise nach zufällig bestimmten Zeitabständen verstanden. Ebenso kann die Durchführung der Testroutine regelmässig ausgelöst werden, wenn bestimmte Umgebungsparameter erfüllt werden.

Gemäss einer ersten Ausführungsform der Erfindung wirkt der Testschaltkreis derart mit den Kommunikationsmitteln zusammen, dass nach jeder erfolgten Testroutine eine Bestätigung an den Empfänger übermittelt wird.

Auf diese Weise kann der Empfänger zentral überprüfen, ob die Testroutine in einem Gerät bzw. in jedem Gerät einer Mehrzahl von Geräten durchgeführt worden ist. Wenn die Bestätigung des Durchführens einer Testroutine fehlt, muss davon ausgegangen werden, dass im Gerät ein grundlegender Defekt besteht. Ebenso muss davon ausgegangen werden, dass im Gerät ein grundlegender Defekt besteht, wenn bei einem Anruf von der Zentrale die Vorrichtung nicht antwortet. In diesem Fall kann ein Servicetechniker mit der Kontrolle des Gerätes beauftragt werden. Die Überprüfung des Ablaufs der Testroutine kann beim Empfänger selbstverständlich automatisch erfolgen. Die Übermittlung der Bestätigung des Durchführens der Testroutine kann beispielsweise als SMS (Short Message) übermittelt werden. Falls innerhalb eines vorbestimmbaren Zeitraums der Empfang einer SMS beim Empfänger unterbleibt, können beim Empfänger automatisch gewisse Aktionen ausgelöst werden, beispielsweise die Alarmierung von verantwortlichen Personen.

Gemäss einer weiteren Ausführungsform der Erfindung ist die Prüfschaltung, d.h. beispielsweise der Testschaltkreis derart mit den Kommunikationsmittels gekoppelt, dass beim Feststellen eines Fehlers in der Testroutine eine Warnmeldung an den Empfänger übermittelt wird. Gleichzeitig mit der Warnmeldung kann insbesondere auch ein Fehlerprotokoll übermittelt werden. Dadurch ist sichergestellt, dass der Empfänger einerseits über Probleme des Gerätes informiert ist. Andererseits kann aufgrund des Fehlerprotokolls gegebenenfalls bereits auf die Fehlerquelle geschlossen werden und der Servicetechniker kann geeignete Ersatzteile mit sich nehmen.

Gemäss einer weiteren bevorzugten Ausführungsform der Erfindung ist die Vorrichtung mit einem Lautsprecher und/oder mit einem Mikrophon versehen. Der Lautsprecher und/oder das Mikrophon sind mit den Kommunikationsmitteln gekoppelt. Sie können zur Übermittlung von Sprache dienen. Die Kommunikationsmittel können also auch dazu dienen, durch Sprachübermittlung medizinisch nicht geschultes Personal bei der Durchführung der Elektrotherapie zu unterstützen. Die Sprachverbindung kann beispielsweise automatisch eingestellt werden, sobald das Gerät in Betrieb genommen wird.

Die Vorrichtung kann ausserdem mit Mitteln zum Erfassen des Status einer Energiequelle versehen sein. Als Energiequellen kommen typischerweise Batterien oder Akkumulatoren zum Einsatz, die im Laufe ihrer Lebenszeit auch ohne Betrieb der Vorrichtung ihre Kapazität reduzieren. Ausserdem benötigt auch der Ablauf der Testroutine eine gewisse elektrische Energie. Die Messmittel zum Erfassen des Status der Energiequelle sind ebenfalls mit den Kommunikationsmittel gekoppelt. Sobald der Status der Energiequelle nicht mehr ausreichend ist, kann über die Kommunikationsmittel ebenfalls eine Warnmeldung an den Empfänger übermittelt werden. Die Kontrolle der Messmittel kann über die Prüfschaltung erfolgen. Es ist aber auch denkbar, den Status der Energiequelle unabhängig zu überprüfen und allfällige Fehler unabhängig zu übermitteln.

Die Kommunikationsmittel können ausserdem auch zum Empfangen von Daten ausgebildet sein. Dies kann insbesondere auch zur Fernwartung genutzt werden. Falls sich aus dem Fehlerprotokoll als Fehlerursache Softwarefehler entnehmen lassen, können diese gegebenenfalls durch Fernwartung behoben werden. Ausserdem ist es auch denkbar, neue Softwarereleases über die Kommunikationsmittel im Gerät einzuspielen. Dazu können die Kommunikationsmittel mit der Rechneranordnung und/oder der Prüfschaltung gekoppelt werden.

Gemäss einer weiteren bevorzugten Ausführungsform der Erfindung kann die Vorrichtung derart ausgebildet sein, dass dem Empfänger nach jeder Benutzung der Vorrichtung mit den Kommunikationsmitteln eine Bestätigung übermittelt wird. Dies kann einerseits zur Erfassung von statistischen Angaben betreffend die Häufigkeit der Benutzung dienen. Nach einer Benutzung sollte ein solches Gerät jeweils gewartet werden. Die Bestätigung kann also dazu dienen, einen Servicetechniker mit der Wartung zu beauftragen. Ausserdem kann die Vorrichtung derart eingerichtet sein, dass nach Inbetriebnahme der Vorrichtung dem Empfänger oder einem weiteren, vorbestimmbaren zweiten Empfänger eine Alarmmitteilung übermittelt wird. Moderne Geräte können zwar wie ausgeführt durch medizinisch ungeschultes Personal eingesetzt werden. Es ist aber trotzdem wichtig, dass ein Patient sich anschliessend in ärztliche Behandlung begibt. Daher ist es vorteilhaft, wenn automatisch bei Inbetriebnahme der Vorrichtung beispielsweise ein Rettungssanitäter alarmiert wird.

Es ist ausserdem denkbar, die erfindungsgemässe Vorrichtung mit Montagemitteln zum Befestigen beispielsweise an einer Wand zu versehen. Die Vorrichtung kann in diesem Fall Sensormittel aufweisen, welche die Anwesenheit der Vorrichtung in den Montagemitteln erfassen. Die Kommunikationsmittel können dabei derart ausgebildet sein, dass eine Warnmeldung übermittelt wird, sobald die Vorrichtung aus den Montagemitteln entnommen wird. Auf diese Weise kann beispielsweise festgestellt werden, wenn die Vorrichtung entwendet wurde. Es ist ausserdem denkbar, die Vorrichtung mit einem GPS Modul zu versehen. Ein in die Vorrichtung integriertes GPS Modul hat verschiedene Vorteile. Wenn das Gerät entwendet wurde, kann die Position des Gerätes ermittelt werden. Wenn das Gerät in einem Noteinsatz ist, kann auf Grund des GPS Moduls der Ort des Einsatzes ermittelt werden. Auf diese Weise können Rettungssanitäter zielgerichtet geleitet werden. Das GPS Modul kann daher typischerweise in Betrieb gesetzt werden, wenn die Vorrichtung aus der Halterung entnommen wird oder wenn die Vorrichtung in Betrieb genommen wird.

Das GSM Modul kann wahlweise in der Vorrichtung aber auch in der Halterung angeordnet sein. Eine Anordnung des GSM Moduls in der Halterung weist den Vorteil auf, dass das GSM Modul über eine Stromversorgung versorgt werden kann, so dass keine Batterien in der Vorrichtung beansprucht werden.

Das erfindungsgemässe Verfahren zum Testen und/oder Betreiben einer Vorrichtung zur Elektrotherapie wird vorzugsweise mit einer wie vorstehend beschriebenen Vorrichtung durchgeführt. In einem ersten Schritt des Verfahrens wird regelmässig eine Testroutine durchgeführt. Die Durchführung kann periodisch oder aufgrund von anderen Regeln erfolgen. Beim Durchführen der Testroutine wird überprüft, ob ein vorbestimmbares Testkriterium erfüllt wird. Wenn das Testkriterium erfüllt wird, werden an einen Empfänger Informationen über in der Vorrichtung enthaltene Kommunikationsmittel übermittelt. Es ist auch denkbar, Informationen dann zu übermitteln, wenn das Testkriterium nicht erfüllt wird.

Zum Überprüfen des Testkriteriums kann beispielsweise geprüft werden, ob eine oder mehrere der folgenden Bedingungen erfüllt sind:
- Wurde eine Testroutine ausgeführt?
- Wurde in der Testroutine ein Fehler aufgefunden?
- Ist der Batteriestatus nicht mehr ausreichend?
- Wurde die Vorrichtung benutzt?/Ist die Vorrichtung in Betrieb?
- Wurde die Vorrichtung aus Montagemitteln entnommen?

Die verschiedenen Testkriterien können mit verschiedenen Häufigkeiten durchgeführt werden. So kann es beispielsweise notwendig sein, die Anwesenheit der Vorrichtung in einer Halterung häufiger zu prüfen. Testroutinen brauchen aber nur ca. alle zwei Wochen durchgeführt zu werden. Wenn geprüft wird, ob die Vorrichtung in Betrieb genommen wird oder entfernt wurde, sollte eine Alarmierung rasch erfolgen. In diesem Zusammenhang ist es daher bevorzugt, eine Alarmmeldung zu übermitteln, sobald der Benutzer einen Einschaltknopf betätigt hat und/oder sobald die Vorrichtung aus der Halterung entnommen wird.

Die erfindungsgemässe Vorrichtung kann im Rahmen eines Gesamtsystems eingesetzt werden, welches aus einer zentralen Verwaltungsstelle und einer Mehrzahl von solchen Geräten zur Elektrotherapie besteht. Die zentrale Erfassungsstelle ist zum Empfang und zur Auswertung der von allen Vorrichtungen übermittelten Daten ausgebildet.

Die Erfindung wird im Folgenden anhand der Figuren und in Ausführungsbeispielen näher erläutert. Es zeigen:

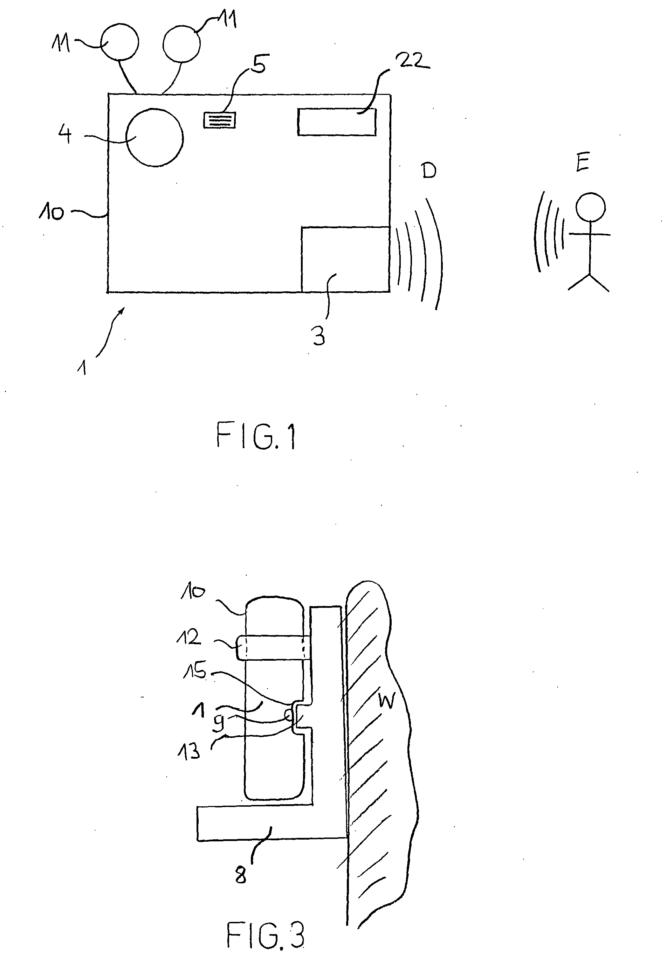

- Figur 1:: eine schematische Darstellung einer erfindungsgemässen Vorrichtung,
- Figur 2:: eine schematische Darstellung der verschiedenen Komponenten einer erfindungsgemässen Vorrichtung,
- Figur 3:: eine schematische Darstellung einer Halterung für die erfindungsgemässe Vorrichtung,
- Figur 4:: ein Flussdiagramm für den Betrieb der erfindungsgemässen Vorrichtung,
- Figur 5:: eine schematische Darstellung eines Systems mit einer Mehrzahl von erfindungsgemässen Vorrichtungen und
- Figur 6:: eine schematische Darstellung eines alternativen Ausführungsbeispiels einer Halterung für die erfindungsgemässe Vorrichtung.

In Figur ist eine Vorrichtung 1 zur Ausübung einer Elektrotherapie dargestellt. Die Vorrichtung 1 ist typischerweise als tragbarer Defibrillator ausgebildet. Die Vorrichtung 1 ist in an sich bekannter Weise mit Elektroden 11 zur Abgabe von elektrischer Energie an einem Patienten versehen. Die Vorrichtung 1 weist im Übrigen weitere Bauelemente herkömmlicher Defibrillatoren auf, welche nicht im Detail gezeigt sind.

Die Vorrichtung 1 ist mit einem GSM Modul 3 versehen. Das GSM Modul 3 dient zur Übermittlung von Daten D an einen Empfänger E. Der Empfänger E kann beispielsweise der Hersteller der Vorrichtung 1 oder ein mit deren Wartung beauftragtes Unternehmen sein. Der Empfänger E kann aber auch ein Krankenhaus, ein Arzt, ein Rettungssanitäter oder eine andere Organisation wie beispielsweise Berufsfeuerwehren oder Betriebsfeuerwehren sein.

Das Gerät ist ausserdem mit einem Lautsprecher 4 und einem Mikrophon 5 versehen. Der Lautsprecher 4 und das Mikrophon 5 sind in nicht dargestellter Weise mit dem GSM Modul 3 verbunden. Das Mikrophon 5 und der Lautsprecher 4 ermöglichen eine Sprachkommunikation zwischen dem Benutzer der Vorrichtung 1 und dem Empfänger E. Die Vorrichtung ist ausserdem mit Bedienelementen 22 versehen. Die Bedienelemente 22 bestehen typischerweise aus einem Ein-/Ausschaltknopf und aus Mitteln zum Auslösen der Therapie.

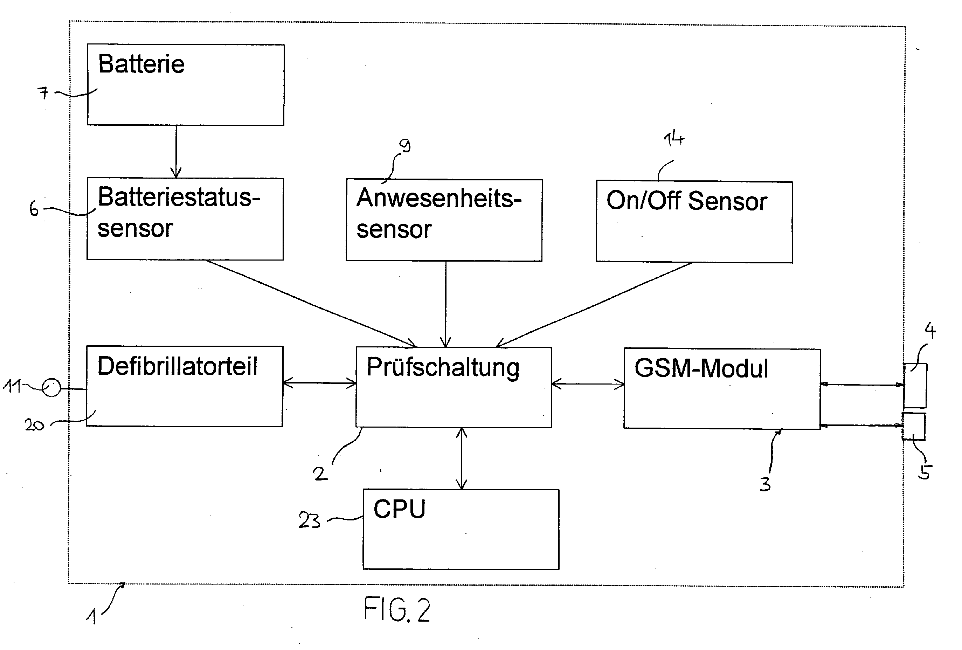

Die Vorrichtung 1 ist in einem Gehäuse 10 angeordnet, welches für die Verwendung als tragbares Gerät ausreichend stabil ist. Figur 2 zeigt im Rahmen der Erfindung wesentliche Komponenten der Vorrichtung 1. Die Vorrichtung 1 wird von einer Rechneranordnung 23 betrieben. Die Rechneranordnung 23 ist ein handelsüblicher Prozessor, welcher gegebenenfalls mit geeigneten Speichern versehen ist. Die Vorrichtung 1 weist insbesondere eine Prüfschaltung 2 auf. Die Prüfschaltung 2 dient zum Überprüfen von einem oder mehreren Testkriterien. In ersten Linie dient die Prüfschaltung 2 zum regelmässigen, insbesondere periodischen Durchführen einer Testroutine des Defibrillatorteils 20. Der Defibrillatorteil 20 ist auf herkömmliche Art und Weise ausgebildet und weist Elektroden 11 auf. Die Prüfschaltung 2 führt eine ebenfalls im wesentlichen bekannte Testroutine durch, bei dem die wesentlichen Teile des Defibrillatorteils 20 und des Prozessor 23 geprüft werden können. Im Rahmen der Erfindung ist es möglich aber nicht zwingend, über die Elektroden 11 elektrische Energie abzugeben.

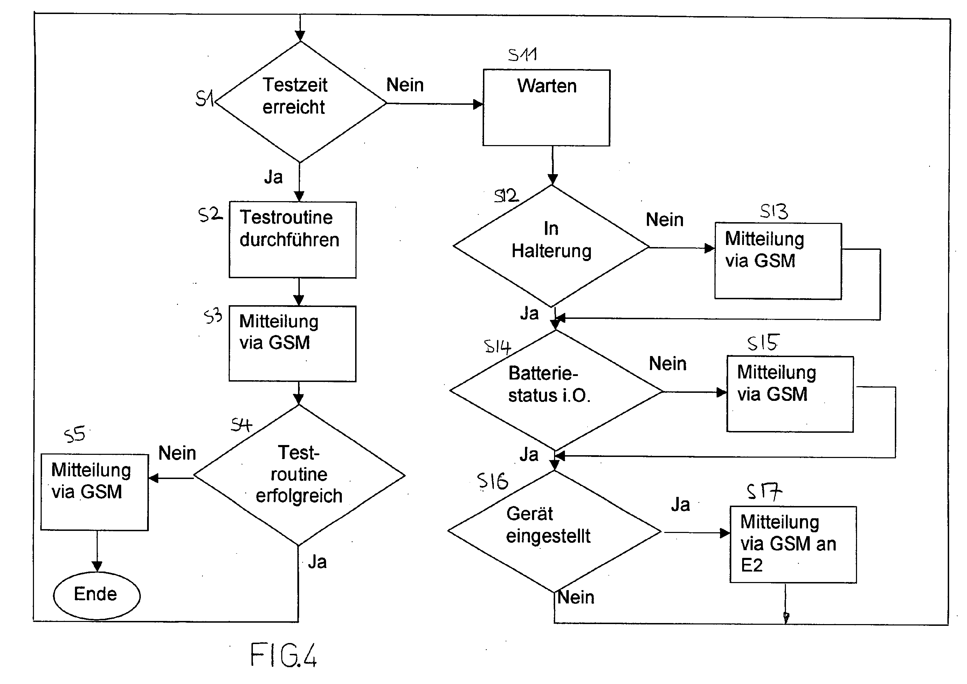

Die Prüfschaltung 2 kann ausserdem zum Überprüfen von verschiedenen weiteren Kriterien vorgesehen sein. Die Vorrichtung 1 wird typischerweise mit einer Batterie 7 betrieben. Die Prüfschaltung 2 prüft mittels einem Batteriestatussensor 6, ob der Status der Batterie noch ausreichend hoch ist. Die Prüfschaltung kann ausserdem über einen Anwesenheitssensor 9 prüfen, ob die Vorrichtung 1 noch in einer Halterung 8 (siehe Figur 3) gehalten ist. Ausserdem kann die Prüfschaltung mit einem On-/Offsensor 14 überprüfen, ob die Vorrichtung 1 in Betrieb ist.

Sobald mit der Prüfschaltung festgestellt wird, dass ein bestimmtes Testkriterium erfüllt wird, wird über ein GSM Modul 3 eine entsprechende Information an den Empfänger E übermittelt. Es ist auch denkbar, gewisse Informationen unmittelbar und ohne Verwendung der Prüfschaltung zu übermitteln. So kann beispielsweise beim Einschalten des Gerätes automatisch eine Mitteilung über das GSM Modul 3 übermittelt werden.

Direkt mit dem GSM Modul 3 ist ein Lautsprecher 4 und ein Mikrophon 5 verbunden. Unabhängig vom übrigen Betrieb der Vorrichtung 1 dienen der Lautsprecher 4 und das Mikrophon 5 in an sich bekannter Weise zum Übermitteln von Sprachinformationen an den Empfänger E.

Figur 3 zeigt schematisch die Befestigung einer erfindungsgemässen Vorrichtung 1 beispielsweise an einer Wand W eines öffentlich zugänglichen Gebäudes. An der Wand ist eine Halterung 8 vorgesehen, in welche die Vorrichtung 1 gestellt werden kann. Die Vorrichtung 1 ist mittels eines Befestigungsbandes 12 in der Halterung 8 gehalten. Die Halterung 8 weist einen Vorsprung 13 auf, der den Anwesenheitssensor 9 in einer Aussparung 15 des Gehäuses 10 mechanisch betätigt. Es sind aber auch andere Anwesenheitssensoren, beispielsweise durch elektrischen Kontakt oder optische Sensoren denkbar. Sobald die Vorrichtung 1 aus der Halterung 8 entnommen wird, wird der Anwesenheitssensor 9 deaktiviert oder aktiviert. Damit kann über das GSM Modul 3 eine entsprechende Information an den Empfänger E oder an einen weiteren Empfänger übermittelt werden.

In Figur 4 ist ein typischer Ablauf des Testverfahrens gezeigt, welches mit der erfindungsgemässen Vorrichtung durchgeführt wird. In einem ersten Testschritt s1 wird geprüft, ob eine Testzeit erreicht worden ist. Dazu wird auf an sich bekannte Weise der Status einer nicht gezeigten Uhr abgefragt und mit einer vorbestimmten Zeitbedingung verglichen. Als Testzeit kann beispielsweise ein Zeitabschnitt zwei Wochen eingegeben werden. Wenn die Testzeit erreicht worden ist, wird in einem Schritt s2 eine Testroutine durchgeführt. Die Testroutine testet den Defibrillatorteil 20 in an sich bekannter Weise. Sobald die Testroutine durchgeführt worden ist, erfolgt in einem Schritt s3 eine Übermittlung einer Mitteilung an den Empfänger E via das GSM Modul 3.

Nach Abschluss der Testroutine wird in einem Schritt s4 geprüft, ob die Testroutine erfolgreich war. Wenn die Testroutine nicht erfolgreich war, wird ein Fehlerprotokoll erstellt und es erfolgt in Schritt s5 eine Mitteilung an den Empfänger E mit dem GSM Modul 3. Gegebenenfalls wird das nicht dargestellte Fehlerprotokoll ebenfalls übermittelt. Nach Übermittlung dieser Information ist der Testablauf beendet. Es muss davon ausgegangen werden, dass die Vorrichtung 1 nicht mehr betriebsfähig ist. Gegebenenfalls kann der Empfänger E versuchen, via Fernwartung über das GSM Modul 3 auf die Vorrichtung 1 zuzugreifen und softwaremässig Reparaturen vorzunehmen. Falls dies gelingt wird die Testprozedur erneut gestartet und es erfolgt eine erneute Prüfung in Schritt s1.

Wenn in Schritt s1 die Testzeit noch nicht erreicht wurde, wird eine vorbestimmbare Zeit gewartet. Nachdem die Zeit in s11 abgelaufen ist, wird in einem ersten Prüfschritt s12 geprüft, ob sich die Vorrichtung 1 in einer Halterung 8 befindet. Die Wartezeit in Schritt s11 kann verhältnismässig kurz eingestellt werden. Wenn sich die Vorrichtung nicht mehr in der Halterung befindet, wird in s13 eine Mitteilung via GSM übermittelt. Der Empfänger E wird dabei informiert, dass die Vorrichtung aus der Halterung 8 entnommen wurde. Anschliessend wird in s14 der Batteriestatus geprüft. Wenn der Batteriestatus nicht in Ordnung ist, erfolgt in s15 ebenfalls eine Übermittlung an den Empfänger E2. Anschliessend wird in s16 geprüft, ob das Gerät in Betrieb ist. Wenn das Gerät eingestellt wurde, erfolgt in s17 eine Übermittlung einer Information an einen weiteren Empfänger E2. Wenn das Gerät nicht eingestellt wurde, wird der Ablauf in s1 neu begonnen.

Es ist auch denkbar, die Testkriterien nach s12, s14, s16 unabhängig voneinander und zu unterschiedlichen Zeiten durchzuführen. Die Durchführung und Verwaltung der einzelnen Test erfolgt dabei vorteilhaft, jedoch nicht zwingend über die in Figur 2 gezeigte Prüfschaltung 2. Typischerweise kann eine Übermittlung einer Information an den Empfänger ausgelöst werden, wenn die Vorrichtung aus der Halterung entnommen wird und wenn das Gerät eingeschaltet wird. Dann kann auf die regelmässige Abfrage in S12 und S16 verzichtet werden.

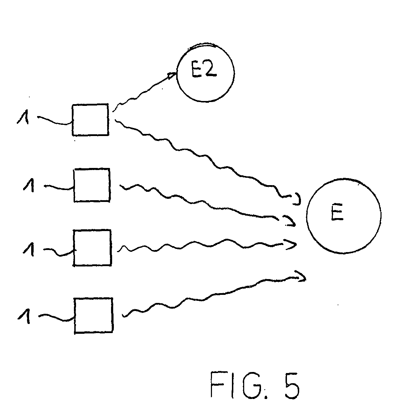

In Figur 5 ist schematisch ein System gemäss der Erfindung gezeigt. Das System besteht aus einer Mehrzahl von Vorrichtung 1 zur Elektrotherapie, welche alle mit einem zentralen Empfänger E kommunizieren. Die einzelnen Vorrichtungen können ausserdem mit einem weiteren Empfänger E2 kommunizieren, wenn spezifische Testkriterien wie beispielsweise die Inbetriebnahme der Vorrichtung 1 erfüllt sind.

Die verschiedenen Geräte 1 werden durch den zentralen Empfänger E verwaltet. Typischerweise werden Angaben über Alter der Batterie, Anzahl der Einsätze, Fehler datenbankmässig erfasst und verwaltet. Sobald von einem der Geräte Informationen übermittelt werden, die eine Kontrolle oder Reparatur eines der Geräte 1 erfordern, kann der Empfänger E reagieren. Dies kann entweder durch Fernwartung über das GSM Modul 3 oder durch Einsatz eines Servicetechnikers erfolgen.

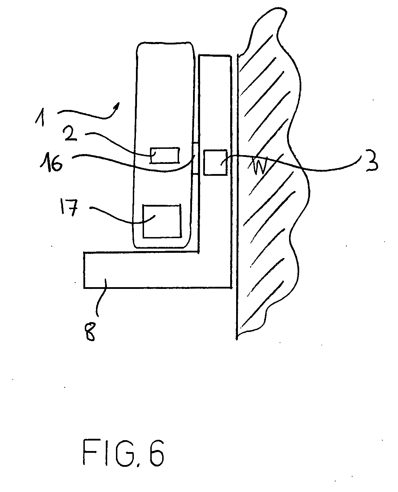

In Figur 6 ist ein alternatives Ausführungsbeispiel einer Halterung 8 gezeigt. Die Halterung ist ähnlich ausgebildet wie die Halterung gemäss Figur 3. Im Unterschied zu Figur 3 ist das GSM Modul 3 jedoch in der Halterung 8 und nicht in der Vorrichtung 1 angeordnet. Eine elektrische Verbindung 16, welche automatisch erstellt wird, wenn die Vorrichtung 1 in die Halterung 8 eingesetzt wird, erlaubt eine Kommunikation zwischen dem GSM Modul 3 und der Vorrichtung 1, insbesondere deren Testschaltkreis 2.

In der Vorrichtung 1 ist ausserdem eine GPS Anordnung 17 vorgesehen. Die GPS Anordnung 17 erlaubt die Lokalisation der Vorrichtung 1, wenn diese aus der Halterung 8 entnommen worden ist. Die GPS Anordnung kann automatisch in Betrieb genommen werden, sobald die Vorrichtung 1 aus der Halterung 8 entfernt wird.

Eine GPS Anordnung ist auch sinnvoll in einem Ausführungsbeispiel wie in Figur 3 gezeigt, wo das GSM Modul 3 in der Vorrichtung 1 angeordnet ist.

Es ist auch denkbar, die Positionsmeldung durch die GPS Anordnung erst zu senden, wenn die Vorrichtung 1 von extern, beispielsweise von der Zentrale angesprochen wird. Beispielsweise kann von der Zentrale eine SMS Mitteilung als Anfrage an die Vorrichtung 1 geschickt werden. Bei Erhalt einer entsprechenden SMS Mitteilung wird die GPS Anordnung in Betrieb genommen und meldet, beispielsweise über SMS die Position der Vorrichtung 1 zurück an die Zentrale.

Die Möglichkeit, SMS zu empfangen kann beispielsweise auch erst aktiviert werden, wenn die Vorrichtung 1 aus der Halterung 8 entnommen wird. Auf diese Weise kann zusätzlich die Batterie in der Vorrichtung 1 geschont werden. Ausserdem ist es denkbar, die Suche des GSM Moduls nach der nächstliegenden, geeigneten GSM Zelle zu unterdrücken, solange sich die Vorrichtung 1 in der Halterung 8 befindet. Wenn die Vorrichtung in der Halterung 8 ist, erfolgt die GSM Verbindung immer über den gleichen, nächstliegenden Sender. Auch auf diese Weise kann der Batterieverbrauch reduziert werden.

## Patentansprüche

1. Vorrichtung (1) zur Elektrotherapie, insbesondere ein tragbarer Defibrillator,
mit einer Prüfschaltung, insbesondere einem Testschaltkreis (2) zum Überprüfen von Funktionen der Vorrichtung in einer Testroutine und/oder zum Ermitteln von Zustandwerten der Vorrichtung,
**dadurch gekennzeichnet, dass** die Vorrichtung Kommunikationsmittel (3) zur Datenübertragung aufweist, welche mit der Prüfschaltung gekoppelt oder koppelbar sind und mittels welcher das Durchführen und/oder Resultate der Testroutine und/oder Zustandwerte der Vorrichtung (1) einem Empfänger (E) übermittelbar sind.
Oder dass die Prüfschaltung (2) darart ausgebildet ist, dass sie auf Grund eines von einer Zentrale übermittelten Befehls die Testroutine ausführt.

2. Vorrichtung nach Anspruch 1, **dadurch gekennzeichnet, dass** die Kommunikationsmittel zur drahtlosen Datenübermittlung ausgebildet sind, insbesondere durch ein GSM Modul gebildet sind.

3. Vorrichtung nach einem der Ansprüche 1 oder 2, **dadurch gekennzeichnet, dass** die Prüfschaltung (2) zum regelmässigen, insbesondere periodischen Durchführen der Testroutine ausgebildet ist.

4. Vorrichtung nach Anspruch 3, **dadurch gekennzeichnet, dass** die Prüfschaltung derart mit den Kommunikationsmitteln (3) zusammenwirkt, dass nach jeder erfolgten Testroutine eine Bestätigung an den Empfänger (E) übermittelt wird.

5. Vorrichtung nach einem der Ansprüche 1 bis 4, **dadurch gekennzeichnet, dass** der Testschaltkreis (2) derart mit den Kommunikationsmitteln (3) zusammenwirkt, dass beim Feststellen eines Fehlers in der Testroutine eine Warnmeldung an den Empfänger (E) übermittelt wird und dass insbesondere ein Fehlerprotokoll übermittelt wird.

6. Vorrichtung nach einem der Ansprüche 1 bis 5, **dadurch gekennzeichnet, dass** die Vorrichtung einen Lautsprecher (4) und /oder ein Mikrophon (5) aufweist, welche mit den Kommunikationsmitteln (3) gekoppelt sind und zum Übertragen von Sprache dienen.

7. Vorrichtung nach einem der Ansprüche 4 bis 6, **dadurch gekennzeichnet, dass** die Vorrichtung mit Messmitteln (6) zum Erfassen des Status einer Energiequelle (7) versehen ist, und dass die Messmittel zum Erfassen des Status der Energiequelle (7) direkt oder indirekt mit den Kommunikationsmittel (3) gekoppelt sind.

8. Vorrichtung nach einem der Ansprüche 1 bis 7, **dadurch gekennzeichnet, dass** die Kommunikationsmittel (3) zum Empfangen von Daten ausgebildet sind und dass die Kommunikationsmittel (3) insbesondere zum Zweck der Fernwartung mit einer Rechneranordnung (23) und/oder der Prüfschaltung (2) und/oder einem Defibrillatorteil (20) gekoppelt oder koppelbar sind.

9. Vorrichtung nach einem der Ansprüche 1 bis 8, **dadurch gekennzeichnet, dass** die Vorrichtung (1) derart ausgebildet ist, dass dem Empfänger (E) nach jeder Benutzung der Vorrichtung (1) mittels der Kommunikationsmittel (3) eine Bestätigung übermittelt wird.

10. Vorrichtung nach einem der Ansprüche 1 bis 9, **dadurch gekennzeichnet, dass** die Vorrichtung derart ausgebildet ist, dass nach Inbetriebnahme der Vorrichtung (1) dem Empfänger (E) oder einem vorbestimmbaren weiteren Empfänger (E2) mittels der Kommunikationsmittel (3) eine Alarmmeldung übermittelt wird.

11. Vorrichtung nach einem der Ansprüche 1 bis 10, **dadurch gekennzeichnet, dass** die Vorrichtung mit Montagemitteln (8) zum Befestigen der Vorrichtung (1) versehen ist, wobei an der Vorrichtung (1) ausserdem Sensormittel (9, 13, 15) vorgesehen sind, welche die Anwesenheit der Vorrichtung in den Montagemitteln (8) erfassen und wobei die Kommunikationsmittel (3) eine Warnmeldung übermitteln, sobald die Vorrichtung (1) aus den Montagemitteln (8) entnommen ist.

12. Verfahren zum Testen und/oder Betreiben einer Vorrichtung (1) zur Elektrotherapie insbesondere einer Vorrichtung nach einem der Ansprüche 1 bis 11, bestehend aus den Schritten
- Regelmässiges Durchführen einer Testroutine;
- Überprüfen, ob ein vorbestimmbares Testkriterium erfüllt ist;
- Übermitteln von Daten an einen Empfänger (E, E2) mittels in der Vorrichtung (1) enthaltenen Kommunikationsmitteln (3) sofern das Testkriterium erfüllt ist.

13. Verfahren nach Anspruch 12, **dadurch gekennzeichnet, dass** zum Überprüfen des Testkriteriums geprüft wird, ob eine oder mehrere der folgenden Bedingungen erfüllt sind:
- Wurde eine Testroutine ausgeführt?
- Wurden in der Testroutine Fehler gefunden?
- Ist der Batteriestatus nicht mehr ausreichend?
- Wird die Vorrichtung benutzt?
- Ist die Vorrichtung aus Montagemitteln (8) entnommen?

14. System mit einer Mehrzahl von Vorrichtungen nach einem der Ansprüche 1 bis 11 und einer zentralen Erfassungsstelle zum Empfang und/oder zur Auswertung von durch die Kommunikationsmittel (3) der Vorrichtungen (1) an die zentrale Erfassungsstelle (E) übermittelten Daten (D).

15. Vorrichtung nach einem der Ansprüche 1 bis 11, **dadurch gekennzeichnet, dass** die Vorrichtung mit einer GPS Anordnung (17) versehen ist.

16. Vorrichtung nach einem der Ansprüche 11 oder 15, **dadurch gekennzeichnet, dass** das GSM Modul (3) in den Montagemitteln (8) enthalten ist.
